# EUROPEAN PATENT SPECIFICATION

(11) **EP 1 291 405 B1**
(45) Date of publication and mention of the grant of the patent: **01.03.2006**
(21) Application number: 01932110.8
(22) Date of filing: 17.05.2001
(51) Int. Cl.: C09K 3/00, C09K 3/16, A41D 31/00, A01N 59/16, C08K 3/08, C08K 3/22, C08L 101/00, B65D 81/24, A61N 5/06, D01F 1/10, C08K 3/00

(54) **COMPOSITION FOR FAR INFRARED IRRADIATION WITH EXCELLENT ANTISTATIC PROPERTY AND FIBER AND TEXTILE PRODUCT BOTH CONTAINING THE SAME**
ZUSAMMENSETZUNG FÜR FERNINFRAROTBESTRAHLUNG MIT EXCELLENTEN ANTISTATISCHEN EIGENSCHAFTEN UND FASER- SOWIE TEXTILPRODUKT BEIDES DIESES ENTHALTEND
COMPOSITION POUR RAYONNEMENT INFRAROUGE LOINTAIN PRESENTANT UNE EXCELLENTE PROPRIETE ANTISTATIQUE ET FIBRE ET PRODUIT TEXTILE LA CONTENANT

(30) Priority: 19.05.2000 JP 2000148770
(43) Date of publication of application: 12.03.2003
(73) Proprietor: Komuro, Toshio, Tokyo 176-0011 (JP)
(72) Inventor: Komuro, Toshio, Tokyo 176-0011 (JP)
(74) Representative: Becker Kurig Straus
(86) International application number: PCT/JP2001/004109
(87) International publication number: WO 2001/088054

(56) References cited:
- JP-A- 3 130 467
- JP-A- 7 252 109
- JP-A- 9 077 553
- JP-A- 10 053 758
- JP-A- 62 184 088
- JP-A- 63 274 660
- US-A- 5 466 526
- US-A- 5 779 950

## Description

### Field of the Invention

The present invention is related to a far infrared radiation composition having excellent static-eliminating properties, which contains silver or a silver compound as an essential component. More particularly, the present invention is related to a far infrared radiation composition having excellent static-eliminating properties, which comprises, as main components, alumina, at least one selected from silica and titanium oxide, at least one selected from platinum, palladium, iridium, rhodium, and compounds thereof, and at least one selected from silver and a silver compound as well as a fiber having incorporated the above composition thereinto and a fiber product using the fiber.

### Background Art

In recent years, as far infrared radiation materials with high efficiency, ceramics are attracting attention. Far infrared rays are electromagnetic waves having a radiation wavelength of several to about 400 µm, and exhibit excellent heating effect and excellent drying effect. For this reason, ceramics and metal materials coated with ceramics have been used as far infrared radiation materials in the heating applications, such as stoves, heaters, and kotatsu, and in addition, they have been employed also in the fields of high-quality food processing since they enable the whole of food to be uniformly heated without heating the surface of the food to an excess extent. Further, fibers having incorporated thereinto or having surfaces coated with a far infrared radiation material have a far infrared effect, and therefore are widely used in bedding, clothing, and underwear.

When a far infrared radiation material is intended for heating or drying a certain substance by far infrared rays therefrom, it is necessary that the far infrared radiation material have a wavelength suited to the absorption range of the substance to be radiated. For example, when a human body is radiated, a far infrared radiation material having a wavelength peak in 8 to 14 µm exhibits a large effect around human body temperatures (from 34 to 37 °C).

Thus, development of far infrared radiation materials having high efficiency capable of emitting far infrared rays in a wide range of wavelengths have been vigorously studied, and, for meeting such a demand, the present inventor has made studies on the invention of a far infrared radiation ceramic powder to which platinum is added (see Japanese Unexamined Patent Publication Nos. 1987 -184088 and 1991-190990).

On the other hand, as the applications of the far infrared radiation ceramic powder, an invention in which a ceramic powder is dispersed and incorporated into a synthetic fiber (see Japanese Unexamined Patent Publication No. 1991-190990), and an invention in which a far infrared radiation powder is mixed into a nylon solution or polyester solution, and the resultant mixture is shaped into a thread-form product by nozzle spraying, and the thread is twisted together with the existing thread material to form a twisted thread for fabric (see Japanese Unexamined Patent Publication Nos. 1991-241025 and 1992-73226) are disclosed.

Each of the far infrared radiation ceramic powders which the present inventors have already invented has a high energy ratio in the wave range of 4 µm or higher and is generally effective in the wave range of from 3 to 12 µm. However, development of far infrared radiation ceramic powder or composition having a wider wave range, which can emit far infrared rays at a higher efficiency and has a radiation performance as uniform as a black body, is being desired.

On the other hand, in accordance with the increase of Western style home environment and the spread of air-cooling and heating equipment, the effects of drying of rooms and static electricity caused by the drying is remarkable. Synthetic resins, such as chemical fibers, are widely used in products for housing and household electrical appliances. These synthetic resins are inexpensive and excellent in durability, and hence, tend to be more widely used. However, such synthetic resins generally have properties such that they are easily charged by friction, and especially under dried conditions, phenomena of shock and deposition of dust due to static electricity are likely to occur, resulting in various problems. For example, it is told that, when a person walks on a carpet, the person is charged with static electricity at 1.000 to 4.000 V, and, when a person wearing clothes made of synthetic fibers stands up from a sofa, the person is charged with static electricity at 3,000 to 5,000 V. When the person in such a state touches an electrical conductor, such as a metallic knob, the static electricity is discharged in a moment and the human body may suffer electric shock, causing disorder and further secondary disaster.

For solving the above-mentioned problems of static electricity, a method is known in which a metallic fiber, which is an electrical conductive material, is incorporated into a fiber. However, this method has disadvantages in that it is difficult to adjust the electric resistance of the resultant fiber, and the fiber is opaque and suffers discoloration during molding. In addition, there is also a method in which an antistatic agent comprised of an organic compound is incorporated into a fiber. However, this method has problems in that the antistatic agent gradually bleeds toward the surface, tackiness and contamination of the surface are caused, and further, deterioration of the static-eliminating properties with time is promoted.

Accordingly, an object of the present invention is to provide a composition having excellent static-eliminating properties which can solve the problems accompanying the antistatic agent containing the above-mentioned metallic fiber or organic compound, especially a composition which can be incorporated into a fiber.

Further, another object of the present invention is to provide a far infrared radiation composition which can uniformly emit far infrared rays in a wide wave range at high efficiency, as compared to the conventionally known far infrared radiation materials. Still further, another object of the present invention is to provide a composition having excellent durability and excellent transparency as well as excellent far infrared emissivity. In addition, another object of the present invention is to provide a fiber and a fiber product having incorporated thereinto the above far infrared radiation composition having excellent static-eliminating properties.

### Disclosure of the Invention

The present invention is a far infrared radiation composition having excellent static-eliminating properties, which comprises (i) alumina, (ii) at least one selected from silica and titanium oxide, (iii) at least one element or one compound selected from platinum, a platinum compound, palladium, a palladium compound, iridium, an iridium compound, rhodium and a rhodium compound, and (iv) at least one selected from silver and a silver compound.

In addition, the present invention is a far infrared radiation composition having excellent static-eliminating properties, which comprises 20 to 60 % by weight of component (1), 40 to 80 % by weight of component (ii), 0.0005 to 0.010 % by weight of component (iii), and 0.1 to 10 % by weight of component (iv).

Further, according to the production method of the present invention, alumina and at least one selected from silica and titanium oxide are individually mixed with platinum or a platinum compound dispersed in a colloidal form to allow the respective particles to have deposited platinum thereon, and the resultant particles having deposited platinum thereon are stirred and mixed, and then, a powder of silver or a silver compound is mixed into the particles to form a far infrared radiation composition having excellent static-eliminating properties.

### Brief Description of the Drawings

Fig. 1 are views showing the structure of a far infrared radiation polyester thread according to the present invention.
Fig. 2 are curves for comparison with respect to the infrared radiation strength between the fibers of the present invention and the conventional fiber. In Fig. 2, numeral 1 denotes Example 1, numeral 2 denotes Example 2, and numeral 3 denotes Comparative Example 1.
Fig. 3 are curves for comparison with respect to the photon emission amount between the fibers of the present invention and the conventional fiber. In Fig. 3, numeral 1 denotes Example 1, numeral 2 denotes Example 2, and numeral 3 denotes Comparative Example 1.

### Best mode for practicing the invention

The present invention will be described in more detail.

In the present invention, it is preferred that component (ii) is titanium oxide. In addition, it is preferred that component (iii) is platinum or a platinum compound.

Further, the present invention includes a far infrared radiation mixture having excellent static-eliminating properties, which comprises 0.1 to 25 % by weight of the above composition and 75 to 99.9 % by weight of a synthetic polymer material, a fiber which comprises the above mixture, and fiber products using the above fiber, including clothing products, bedding products, and packaging products.

Furthermore, the present invention includes a nonwoven elastic spongy structure material or board material which comprises the above composition.

In the present invention, the term "static-eliminating properties" means properties of eliminating static electricity charging a substance, i.e., antistatic properties, and the term "static-eliminating material" means a material having static-eliminating properties.

In addition, in the present invention, the fibers include a thread (filament, staple), a hollow fiber, a woven fabric, a knit, and a nonwoven fabric.

Further, in the present invention, the "% by weight" of the sum of the substances constituting the composition or mixture is naturally 100 %.

It is preferred that the composition of the present invention is used in the form of powder, but the form of the composition is not limited to this, and can be in various forms depending on the product applied. For example, the composition in a film form, a layer form, or a powder form can be formed on the surface of another material or inserted into another material to form the so-called coated material or laminate material. Alternatively, the composition in a fluid form can be used as a mixture with a synthetic polymer material.

As component (i) "alumina" (Al₂O₃) contained in the composition of the present invention, it is preferred to use high purity alumina (aluminum oxide) having excellent sinter properties and having a purity of 99.9 % or higher. As the alumina, commercially available powdery high-purity alumina can be used. The content of component (i) is 20 to 60 % by weight, preferably 30 to 50 % by weight. The particle diameter of component (i) depends on the product and form in which the composition of the present invention is used. When the composition in the form of powder is applied to a radiation heating apparatus, such as a far infrared radiation heater, a powder having a general particle diameter, for example, a particle diameter of about several micrometer can be used. However, when the composition in the form of powder is incorporated into a fiber, the particle diameter of component (i) is advantageously adjusted to 2 µm or less in maximum, preferably 1.5 µm or less in maximum, more preferably 1.0 µm or less in maximum, depending on the fiber diameter. The reason for this resides in that, depending on the fiber diameter, there is a danger that clogging of the spinning machine occurs in the spinning step, that a failure is caused to cross-sectional shape of a fiber, and that the fiber is cut in which starting point of the cut being a powder.

In the present invention, as the "silica" (SiO₂), high purity silica having a purity of 99.8 % or higher is preferred, and, for example, commercially available ultrafine anhydrous silica can be used. The particle diameter of the silica is similar to that of component (i). The content of the silica in the composition of the present invention is 40 to 80 % by weight, preferably 50 to 70 % by weight.

In the present invention, with respect to the "titanium oxide" (TiO₂), the purity, the particle diameter, and the amount incorporated are similar to those of the above-mentioned component (i). As the titanium oxide used, for example, rutile and/or anatase titanium dioxide or a mixture thereof is preferred, and more preferred is anatase titanium dioxide. Commercially available ultrafine anhydrous titanium oxide can be used. Alternatively, high-purity finely divided titanium dioxide obtained by granulating and purifying titanium dioxide coarse particles having a purity of 80 % or higher can be used. Thus, component (ii) which is at least one selected from silica and titanium oxide is contained in the composition of the present invention in an amount of 40 to 80 % by weight.

In the present invention, component (iii) "at least one element or one compound selected from platinum or a platinum compound, palladium or a palladium compound, iridium or an iridium compound, and rhodium or a rhodium compound" is contained in the form of colloid and expected to exhibit the so-called colloidal activity for adsorbing oxygen and hydrogen. Platinum or a platinum compound is preferred. It is desired that component (iii) is contained in the composition of the present invention in an amount of 0.0005 to 0.010 % by weight, preferably 0.001 to 0.004 % by weight, in terms of the metal. In addition, as component (iii), it is preferred to use a colloid having dispersed therein component (iii) [hereinafter, referred to as "component (iii) colloid"] obtained by dispersing component (iii) having a particle diameter of about 0.7 to 4 nm (7 to 40 Å) in, for example, a hydrochloric acid solution as a colloid. As the component (iii) colloid, one which contains 0.1 to 5 % by weight, preferably 0.5 to 2 % by weight, more preferably 0.8 to 1.2 % by weight of component (iii) is used, and, taking into consideration the concentration of component (iii) in the colloid, the colloid is added so that component (iii) is contained in the composition in an amount of 0.0005 to 0.010 % by weight. As a method for preparing the component (iii) colloid, commonly used methods can be used. For example, commercially available platinum colloidal solution containing, for example, 1 % by weight of platinum can be used.

In the present invention, it is preferred that component (iv) "silver or a silver compound" is used in the form of powder, and a commercially available silver powder can be used. It is desired that component (iv) is contained in the composition of the present invention in an amount of 0.1 to 10 % by weight, preferably 0.5 to 5 % by weight, more preferably 0.7 to 2.0 % by weight, in terms of silver.

Further, the composition of the present invention can contain silicon nitride. It is considered that silicon nitride facilitates the action of hydrogen and controls the moving direction of hydrogen ions to a specific direction. However, when the composition contains silicon nitride, it is preferred that the content of silicon nitride is 3 % by weight or less.

Further, in addition to the essential composition of the present invention comprising (i) alumina, (ii) at least one selected from silica and titanium oxide, (iii) at least one element or one compound selected from platinum, a platinum compound, palladium, a palladium compound, iridium, an iridium compound, rhodium and a rhodium compound, (iv) at least one selected from silver and a silver compound, and optionally silicon nitride, the present invention may contain a composition and an impurity which are inevitably contained. In addition, the composition may contain an additive for stabilizing the composition, for example, a binder and an additive for imparting an additional function to the composition. Further, the range of the essential composition contained of the present invention falls in the above-mentioned range, and, the total amount of these is naturally 100 % by weight or less.

In the composition of the present invention, component (i) having a predetermined particle diameter and component (ii) are individually mixed with, for example, a platinum colloid or a platinum compound, so that the platinum colloid having a particle diameter of 0.7 to 4 nm (7 to 40 Å) can be deposited on other particles. In this case, taking into consideration the ratio between component (i) and other components in the composition and the amount of platinum in the colloidal solution, the amount of the platinum colloid added is determined so that a desired platinum amount is satisfied and platinum is deposited on the particles of component (i) and other components. Next, the particles having deposited platinum thereon in a predetermined ratio between the components are stirred and mixed together, and a predetermined amount of a powder of silver or a silver compound is mixed thereinto. Alternatively, the composition can be formed by a method in which a predetermined amount of the platinum colloid is added only to a predetermined amount of alumina particles, and then, silica and titanium oxide are added to the alumina having deposited platinum thereon and stirred and mixed together, and a silver powder is added thereto, and stirred and mixed again.

In addition, the composition of the present invention can be formed by a method in which the platinum colloid is mixed with one or more powder raw materials including component (i), component (ii), and component (iv), the mixture is optionally diluted with a solvent or the like in an amount such that the resultant mixture has a fluidity and can be sprayed, and the resultant mixture is uniformly dispersed by spraying, and then, the mixture is heated at about 50 to 150 °C for about 10 minutes to 1 hour. The powder raw materials which are not initially added can be added in an arbitrary stage. As the solvent for dilution, any solvent can be used as long as it does not interfere with the effect of the composition of the present invention, and examples of solvents include pure water and alcohols. For improving the dispersion properties, conventionally known dispersants can be added.

Further, the composition of the present invention can be mixed with a synthetic polymer material, such as a polymeric compound, to form a master batch. Examples of methods of mixing include a method in which the composition of the present invention is rendered in a powder form and incorporated into a synthetic polymer material, and a method in which the composition is rendered in a dispersion form and mixed with a synthetic polymer material. In such cases, the content of the composition of the present invention in the mixture can be 0.1 to 25 % by weight, and the content of the synthetic polymer material in the mixture can be 75 to 99.9 % by weight.

When a synthetic fiber is obtained by spinning from a single master batch containing the composition of the present invention as such, or when two or more master batches of synthetic polymer material each containing the composition of the present invention are provided, and fibers individually spun from the respective batches are mixed spun to obtain a synthetic fiber, the content of the composition of the present invention in the master batch is 0.1 to 3 % by weight, preferably 0.3 to 1.5 % by weight, and the composition can exhibit excellent effect in a relatively small amount. In addition, the master batch containing the composition of the present invention can be diluted by further adding thereto a synthetic polymer material. The synthetic fiber produced from the master batch and the similar or another fiber which does not contain the composition of the present invention can be mixed spun to dilute the concentration of the composition in the resultant fiber. When the composition is diluted in such a way, it is preferred that the content of the composition of the present invention in the master batch is increased to 3 to 25 %. That is, the preferred content of the composition of the present invention in the master batch varies depending on the mixed spinning ratio of the fiber comprising the composition of the present invention to the mixed spun fiber and the synthetic polymer material used, for example, polyester or polyethylene.

The synthetic polymer materials include nylon, vinylon, ester, acryl, urethane, polyamide, polyester, polyacrylonitrile, polyolefin, and acetate, which can form synthetic fibers.

In addition, if desired, into the master batch can be incorporated, for example, catalysts, such as magnesium oxide, mica, calcium carbonate, and zeolite, and various types of additives, such as a plasticizer, an ultraviolet absorber, filler, a coloring agent, a color-protecting agent, a flame retardant, a bleedout-preventing agent, a stabilizer, a heat resisting agent, and a fluorescent brightener.

With respect to the fiber, a filament or a hollow fiber can be spun from the master batch using a commonly used spinning method, for example, a melt spinning method. In the present invention, before spinning, the composition of the present invention is preliminarily mixed with a synthetic polymer material which is a raw material for fiber. In this instance, the composition of the present invention, for example, in a powder form is strongly fixed to the fiber spun, so that the composition of the present invention can be prevented from peeling off. In addition, by employing such a method, the content of the composition of the present invention can be increased, as compared to that in the conventional method. Further, the thus spun synthetic fiber comprising the composition of the present invention can also be mixed spun with another fiber which does not contain the composition of the present invention, for example, a natural fiber, such as cotton, hemp, silk, or wool, or a synthetic fiber.

Next, the fiber comprising the composition of the present invention or the mixed spun fiber obtained by mixed spinning the above fiber can be processed by sewing into various fiber products. Examples of fiber products which can enjoy the characteristic feature of the composition of the present invention include clothing, such as shirts, underclothes, socks, and panty hose. The composition of the present invention can exhibit static-eliminating properties and far infrared effects, and has excellent thermal effect and can not only facilitate the circulation of the blood but also prevent a human body from being charged, so that clothing free from discomfort caused by discharge occurring when the human body touches a metal or discomfort caused when the clothing is taken off can be provided. In addition, bedding, such as futon, blankets, and pillows, supporters and bandages, and packaging products, such as cover cloth for sofas and chairs, which are produced from the fiber comprising the composition of the present invention, have excellent keeping warm properties due to the excellent far infrared effect and static-eliminating effect, and can suppress dust deposition caused by generation of static electricity, thus offering very sanitary fiber products.

The use of the composition of the present invention is not limited to the above-mentioned fibers and fiber products, but the composition can be used in elastic spongy structure materials and board materials. Examples of elastic spongy structure materials include expanded urethane and reexpanded polyethylene, and they can be used as mattresses, cushion materials, and pad materials for bedding and chairs.

The composition of the present invention can be formed as finely divided powder, and the particle diameter of the powder is preferably 0.1 to 2.0 µm, more preferably 0.2 to 1.0 µm.

### Examples

The following Examples should not be construed as limiting the scope of the present invention, and illustrate representative embodiments.

### Example 1

(a) The particle sizes of commercially available alumina, silica, and titanium oxide were individually adjusted so as to be particle size of 1 µm or less. Then, three portions of 0.5 part by weight of a platinum colloidal solution (manufactured by TANAKA PRECIOUS METALS) containing 1 % of platinum (i.e., 0.005 part by weight, in terms of platinum) were individually mixed into 200 parts by weight of each of the above particles, and the resultant three mixtures were mixed together to prepare a colloidal mixture. Then, to 601.5 parts by weight of the prepared mixture was added 6.0 parts by weight of a silver powder (manufactured by TANAKA PRECIOUS METALS) having a particle diameter of 0.2 to 1.0 µm and an average particle diameter of 0.7 µm. Thus, the weight ratio between the components in the composition in this Example is as follows: alumina: 33.0025 % by weight; silica: 33.0025 % by weight; titanium oxide: 33.0025 % by weight; platinum: 0.0025 % by weight; and silver: 0.99 % by weight.
(b) Into the composition obtained in item (a) above was mixed 5,400 parts by weight of polyester so that the ratio of the composition of the present invention to the polyester became about 1:9 to prepare a resin chip (master batch).
(c) The master batch obtained in item (b) above was spun into a 3-denier thread by a melt spinning method.

### Example 2

The master batch prepared in Example 1 was spun into a 6-denier hollow fiber by a melt spinning method.

### Example 3

A composition was prepared in substantially the same manner as in item (a) in Example 1 except that the weight ratio between the components in the composition was changed to one such that platinum was 0.01 % by weight, silver was 10 % by weight silica 0% by weight, alumina 44.995% by weight and titania 44.995% by weight. The prepared composition was diluted with pure water in an amount such that the resultant composition had fluidity and could be sprayed, and sprayed and uniformly dispersed, and then, the resultant mixture was heated at about 50 to 150 °C for about 10 minutes to 1 hour to form a finely divided powder.

### Example 4

A finely divided powder was formed in substantially the same manner as in Example 3, and that the weight ratio between the components in the composition was changed to one such that platinum was 0.002 % by weight, silver was 1.0 % by weight, silica 0% by weight, alumina 49.449 % by weight and titania 49.449% by weight.

### Example 5

A finely divided powder was formed in substantially the same manner as in Example 1 except that the weight ratio between the components in the composition was changed to one such that platinum was 0.01 % by weight, silver was 10 % by weight, and each of titania, alumina, and silica was 30 % by weight.

### Example 6

A finely divided powder was formed in substantially the same manner as in Example 3, and that the weight ratio between the components in the composition was changed to one such that platinum was 0.0005 % by weight, silver was 0.1 % by weight.

### Comparative Example 1

The particle sizes of commercially available alumina, silica, and titanium oxide were individually adjusted so as to be particle size of 1 µm or less. Then, three portions of 0.5 part by weight of a platinum colloidal solution (manufactured by TANAKA PRECIOUS METALS) containing 1 % of platinum (i.e., 0.005 part by weight, in terms of platinum) were individually mixed into 200 parts by weight of each of the above particles, and the resultant three mixtures were mixed together to prepare a colloidal mixture. Thus, the weight ratio between the components in the composition in this Comparative Example is as follows: alumina: 33.3325 % by weight; silica: 33.3325 % by weight; titanium oxide: 33.3325 % by weight; and platinum: 0.0025 % by weight. Into the composition in this Comparative Example was mixed 5,400 parts by weight of polyester so that the ratio of the composition to the polyester became about 1:9 to prepare a resin chip (master batch). This master batch was spun into a 3-denier thread by a melt spinning method.

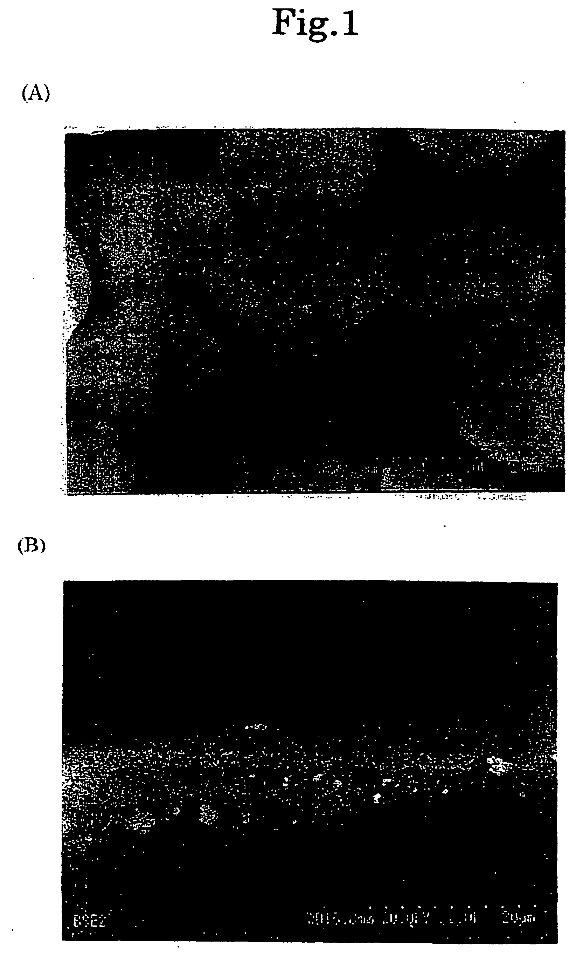

Fig. 1 shows scanning electron microscope (SEM) photomicrographs of the fiber in Example 1. In Fig. 1, (A) is a SEM photomicrograph as observed in a vertical cross-sectional surface of the fiber, and (B) is a SEM photomicrograph as observed in a sidewall surface of the fiber. As a result, it is found that the powder particles in the composition of the present invention are uniformly dispersed and strongly fixed on the surface of the fiber.

Now, the effect of the fiber comprising the composition of the present invention is demonstrated by the infrared emissivity, the photon emission amount, the static-eliminating properties, and the antibacterial and bactericidal properties examination.

### (1) Infrared emissivity

With respect to each of the fibers obtained in Examples 1 and 2 and Comparative Example 1, an infrared emissivity was measured. The infrared emissivity was determined by measuring a spectrum using a spectral dosimeter (model: SR5000, manufactured by IR System Co., Ltd.) having a uniformly heating oven, a surface reflector, and a detector (MCT/InSb). The procedure for measurement is as follows. A sample for measurement was placed on the uniformly heating oven, and divided by partitions and heated so that the infrared rays radiated from the divided samples were polarized in the horizontal direction by the reflector to measure a spectrum. As an infrared standard heat source, the radiation spectrum from a black body oven at the same temperature was used.

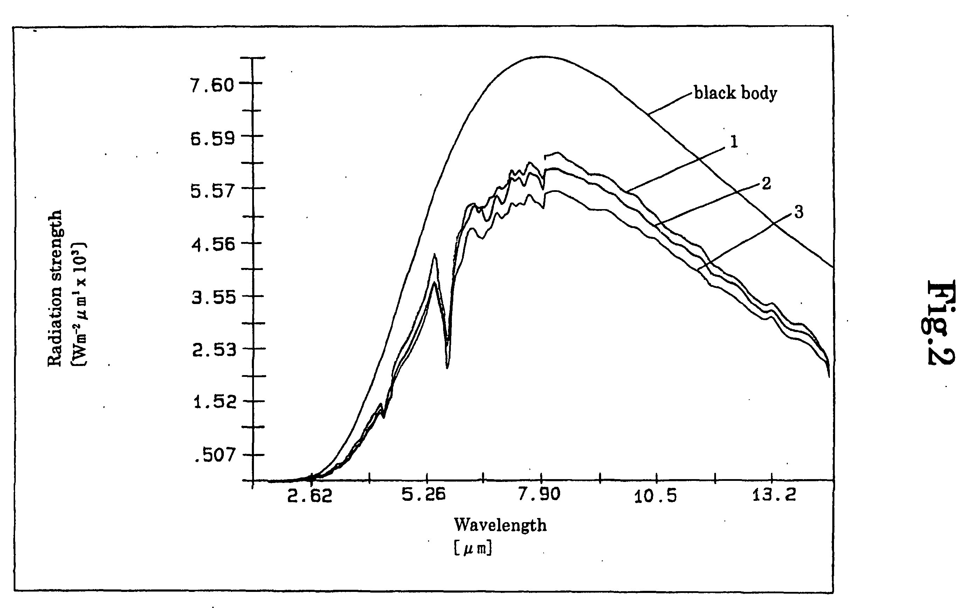

Fig. 2 shows spectral radiation strength spectra in Examples and Comparative Example. From Fig. 2, it is found that the fibers (curves 1 and 2 in Fig. 2) each comprising the composition of the present invention containing a silver powder are excellent in radiation strength in the wave range of from 2.62 to 13.2 µm, as compared to the fiber (curve 3 in Fig. 2) comprising the conventional composition containing no silver powder.

Further, with respect to each of the samples, a radiation spectrum was measured under conditions such that the scanning speed was 4 seconds and the accumulation number was 3. From the radiation spectrum data per wavelength, average emissivities in the wave ranges of from 4 to 12 µm and from 8 to 12 µm were determined by making calculation. The results are shown in Tables 1 and 2.

When comparison is made between the fibers having the same fiber diameter, the fibers of the present invention are more excellent in each of the average emissivities in the wave ranges of from 4 to 12 µm and from 8 to 12 µm by 10 % or more than the conventional fiber.

The infrared emissivities of the powders in Examples 5, 3, 4, and 6 were lowered in this order. The powders stably radiated infrared rays in the wave range of from 8 to 12 µm.

**Table 1: Infrared emissivity of fiber**

| | | | |
|---|---|---|---|
| | Fiber diameter (denier) | Average infrared emissivity | |
| | | 4-12 µm wave range | 8-12 µm wave range |
| Example 1 | 3 | 0.72 | 0.74 |
| Example 2 | 6 (Hollow fiber) | 0.68 | 0.71 |
| Comparative example 1 | 3 | 0.64 | 0.67 |
| Black body | | 0.77 | 0.78 |

**Table 2: Infrared emissivity of powder**

| Example | Formulation (wt%) | | | | | Average infrared emissivity | |
|---|---|---|---|---|---|---|---|
| | Platinum | Silver | Alumina | Silica | Titania | 4-12 µm wave range | 8-12 µm wave range |
| 3 | 0.010 | 10.0 | 44.995 | 0 | 44.995 | 0.84 | 0.91 |
| 4 | 0.002 | 1.0 | 49.499 | 0 | 49.499 | 0.81 | 0.88 |
| 5 | 0.010 | 10.0 | 29.997 | 29.997 | 29.997 | 0.85 | 0.92 |
| 6 | 0.0005 | 0.1 | 44.950 | 0 | 44.950 | 0.81 | 0.87 |

| | | | | | | | |
|---|---|---|---|---|---|---|---|
| Note that, in the column entitled "Formulation" in the above table, each value is rounded to five significant figures, and thus the total number may not be 100. | | | | | | | |

### (2) Photon emission amount

A photon amount was measured using Chemiluminescence Analyzer (model: CLD-110, manufactured by Tohoku Electronic Industrial Co., Ltd.). The measurement was made with respect to 0.5 g of the fiber in each of Examples 1 and 2 and Comparative Example 1 at 100 °C. The procedure for measurement is as follows. Each sample was weighed and placed on a 50 mm φ stainless steel petri dish, and a mask plate prepared by making a 20 mm φ hole in the center of an about 50 mm φ stainless steel plate was placed on the above sample, and the sample was permitted to emit at a gate time of 10 seconds for 20 minutes to measure a photon emission amount.

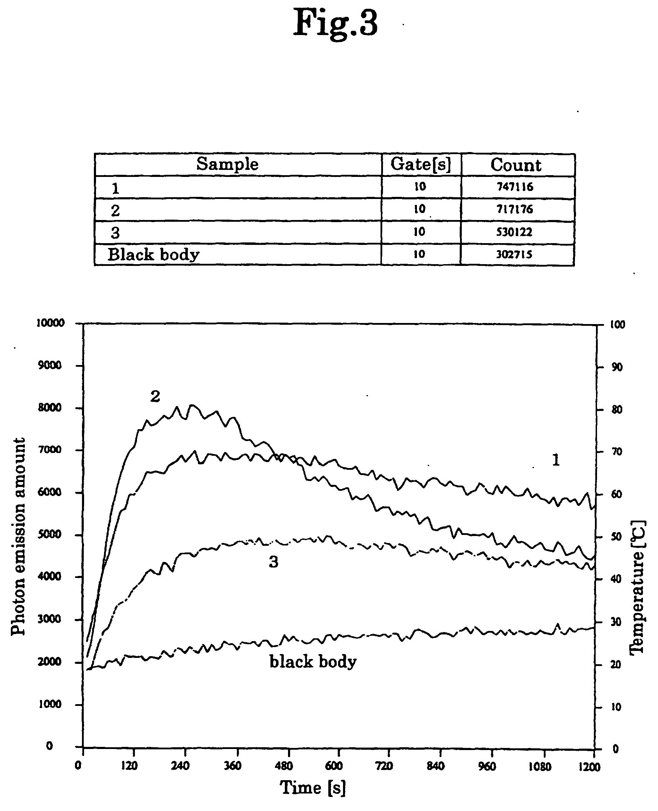

Fig. 3 shows photon emission amounts with the lapse of time, and the photon emission amounts in Examples 1 and 2 are generally large, and especially the emission amounts at the initial stage are large, as compared to that in Comparative Example 1, clearly indicating that the absorbed photons are amplified and emitted at high efficiency in Examples.

The photon emission amounts of powders are shown in Table 3. The powders in the present invention conducted stable photon emission. The photon emission amounts of the powders are in an order such that Example 4 was the highest, then the next came Examples 3 and 5, and the last came Example 6.

**Table 3: Photon emission amount of powder**

| Example | Photon emission amount |
|---|---|
| | (× 10³ counts) |
| 3 | 4718 |
| 4 | 4829 |
| 5 | 4125 |
| 6 | 3181 |

### (3) Charge and static-eliminating properties

Using the fiber in each of Example 1 and Comparative Example 1, knit bear-plain knitted was prepared from two pairs of 75-denier polyester yarn/36 filament, and charge properties was examined in accordance with 5.1A method (Half-life period measurement method) in "Charge examination method for fabric and knit" (JIS L1094-1997). As shown in Table 4, the half-life period, i.e., the time required until the charged voltage was attenuated to 1/2 of the initial voltage, of the knit comprising the fiber of the present invention (Example 1) is 1/10 or less of the knit comprising the fiber in Comparative Example 1. That is, it is found that the knit comprising the fiber of the present invention is very excellent in static-eliminating properties.

**Table 4: Comparison in voltage half-life period between fibers**

| | Knit comprising fiber in Example 1 | Knit comprising fiber in Comparative Example 1 |
|---|---|---|
| Half-life period(s) | 1.19 | 14.02 |

### (4) Antibacterial and bactericidal properties

The composition of the present invention contains silver or a silver compound, and hence, is expected to exhibit an antibacterial effect. Thus, antibacterial and bactericidal properties were examined by the following method.

Staphylococcus aureus (IFO 12732) was used as a bacterium under test, and implanted in nutrient broth and cultured at 28 °C for 24 hours. 0.4 ml of the solution 10-fold diluted with sterilized water was implanted in 20 ml of nutrient broth and shake-cultured at 37 °C for 3 hours. The resultant bacteria liquid was diluted with 1/20 concentration nutrient broth containing 0.05 % Tween 80. 0.4 g of the fiber to be examined was placed in a vial with threaded top having a capacity of 30 ml and sterilized, and then, dried in a clean bench for 60 minutes. On the other hand, as a control, gauze treated in the same manner as in the above fiber examined was used. Next, 0.2 ml of the test bacteria suspension was implanted in the fiber which was a test specimen, and the vial containing the resultant fiber was sealed up with a cap. After completion of the culturing, 20 ml of physiological saline containing 0.2 Tween 80 was added to the vial, and the vial was shaken by hand (shaking width: 30 cm; shaking frequency: 30) so that the bacteria adhering to the test specimen was uniformly dispersed, and than, a viable count in the vial was measured by a pour plate culture method using a nutrient agar medium. The implanted bacteria count was measured in the same manner immediately after the test bacteria suspension was implanted in the control. The results for measurements are shown in Table 5. The bacteria count measured after 18 hours from the start of the culturing in each of the fibers of the present invention (Examples 1 and 2) is remarkably reduced, as compared to that in the fiber in Comparative Example 1, and, as a result, it is found that the fibers of the present invention exhibit excellent antibacterial and bactericidal effects (bacteriostasis activity value, and bactericidal activity value).

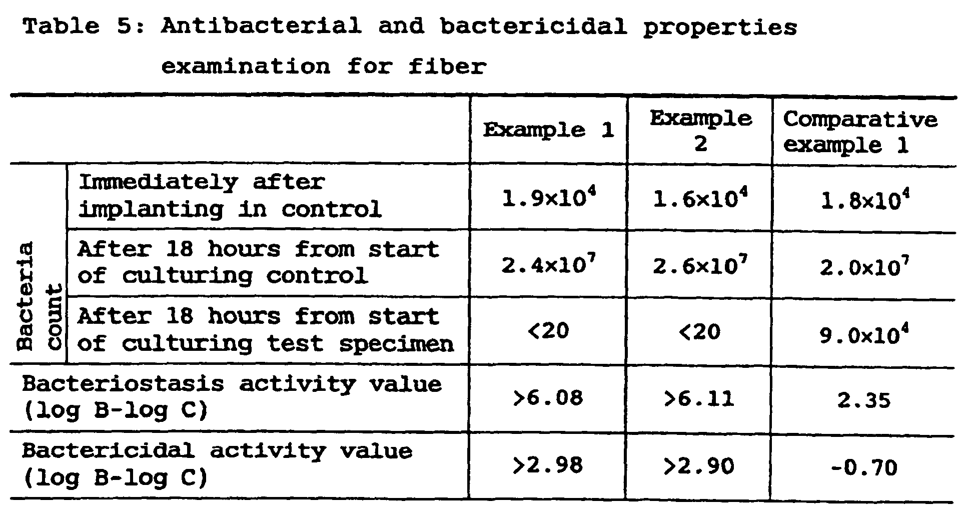

### Industrial Applicability

The composition of the present invention, which comprises alumina, at least one of silica and titanium oxide, at least one of platinum, palladium, iridium, rhodium, and compounds thereof, and at least one of silver and a silver compound, can not only uniformly emit far infrared rays in a wide wave range at high efficiency, but also emit photons in a large amount, as compared to the conventional composition which does not contain at least one of silver and a silver compound. In addition, the composition of the present invention has excellent durability and excellent transparency as well as excellent static-eliminating properties. The fiber obtained by spinning a mixture of the above composition and a synthetic polymer material can exhibit excellent far infrared effect and excellent static-eliminating effect as well as excellent antibacterial and bactericidal effects.

## Claims

1. A far infrared radiation composition having excellent static-eliminating properties, said composition comprising (i) alumina, (ii) at least one selected from silica and titanium oxide, (iii) at least one element or one compound selected from platinum, a platinum compound, palladium, a palladium compound, iridium, an iridium compound, rhodium and a rhodium compound, and (iv) at least one selected from silver and a silver compound.

2. A far infrared radiation composition having excellent static-eliminating properties according to claim 1, which comprises 20 to 60 % by weight of said component (i), 40 to 80 % by weight of said component (ii), 0.0005 to 0.010 % by weight of said component (iii), and 0.1 to 10 % by weight of said component (iv).

3. A far infrared radiation composition having excellent static-eliminating properties according to claim 1 or 2, wherein said component (ii) is titanium oxide.

4. A far infrared radiation composition having excellent static-eliminating properties according to any one of claims 1 to 3, wherein said component (iii) is platinum or a platinum compound.

5. A far infrared radiation material having excellent static-eliminating properties, said material comprising the composition according to any one of claims 1 to 4 which is in the form of powder.

6. A material having excellent static-eliminating properties and excellent far infrared radiation properties, which is fixed on a surface thereof or in internal voids thereof the composition according to any one of claims 1 to 5.

7. A far infrared radiation mixture having excellent static-eliminating properties, said mixture containing:
(A) 0.1 to 25 % by weight of the composition according to any one of claims 1 to 5; and
(B) 75 to 99.9 % by weight of a synthetic polymer material.

8. A synthetic fiber comprising the mixture according to claim 7.

9. An elastic spongy structure material or board material comprising the mixture according to claim 7.

10. A clothing product comprising the synthetic fiber according to claim 8.

11. A bedding product comprising the synthetic fiber according to claim 8.

12. A packaging product comprising the synthetic fiber according to claim 8.

13. A method for producing a far infrared radiation composition having excellent static-eliminating properties, said method comprising: mixing alumina and at least one selected from silica and titanium oxide individually with platinum or a platinum compound dispersed in a colloidal form to allow the respective particles to have deposited platinum thereon; stirring and mixing the resultant particles having deposited platinum thereon; and then mixing into the powder of silver or a silver compound to form a composition.

## Patentansprüche

1. Ferninfrarot-Strahlungszusammensetzung mit hervorragenden aufladungseliminierenden Eigenschaften, wobei die Zusammensetzung umfasst, (i) Aluminiumoxid, (ii) mindestens eines ausgewählt aus Siliziumoxid und Titanoxid, (iii) mindestens ein Element oder eine Verbindung ausgewählt aus Platin, einer Platin-Verbindung, Palladium, einer Palladium-Verbindung, Iridium, einer Iridium-Verbindung, Rhodium und einer Rhodium-Verbindung, und (iv) mindestens eines ausgewählt aus Silber und einer Silber-Verbindung.

2. Ferninfrarot-Strahlungszusammensetzung mit hervorragenden aufladungseliminierenden Eigenschaften nach Anspruch 1, welche umfasst, 20 bis 60 Gew.-% der Komponente (i), 40 bis 80 Gew.-% der Komponente (ii), 0,0005 bis 0,010 Gew.-% der Komponente (iii) und 0,1 bis 10 Gew.-% der Komponente (iv).

3. Ferninfrarot-Strahlungszusammensetzung mit hervorragenden aufladungseliminierenden Eigenschaften nach Anspruch 1 oder 2, worin die Komponente (ii) Titanoxid ist.

4. Ferninfrarot-Strahlungszusammensetzung mit hervorragenden aufladungseliminierenden Eigenschaften nach einem der Ansprüche 1 bis 3, worin die Komponente (iii) Platin oder eine Platin-Verbindung ist.

5. Ferninfrarot-Strahlungsmaterial mit hervorragenden aufladungseliminierenden Eigenschaften, wobei das Material die Zusammensetzung nach einem der Ansprüche 1 bis 4 umfasst, die in der Form eines Pulvers ist.

6. Material mit hervorragenden aufladungseliminierenden Eigenschaften und hervorragenden Ferninfrarot-Strahlungseigenschaften, welches auf einer Oberfläche hiervon oder in Innenräumen hiervon angebracht ist die Zusammensetzung nach einem der Ansprüche 1 bis 5 bzw. worin die Zusammensetzung nach einem der Ansprüche 1 bis 5 auf einer Oberfläche hiervon oder in Innenräumen hiervon angebracht ist.

7. Ferninfrarot-Strahlungsgemisch mit hervorragenden aufladungseliminierenden Eigenschaften, wobei das Gemisch enthält:
(A) 0,1 bis 25 Gew.-% der Zusammensetzung nach einem der Ansprüche 1 bis 5; und
(B) 75 bis 99,9 Gew.-% eines synthetischen Polymermaterials.

8. Synthetische Faser, welche das Gemisch nach Anspruch 7 umfasst.

9. Elastisches Schwammstrukturmaterial oder Platten- bzw. Brett- bzw. Karton-Material, welches das Gemisch nach Anspruch 7 umfasst.

10. Bekleidungsprodukt, welches die synthetische Faser nach Anspruch 8 umfasst.

11. Bettzeugprodukt bzw. Matratzenprodukt, welches die synthetische Faser nach Anspruch 8 umfasst.

12. Verpackungsprodukt, welches die synthetische Faser nach Anspruch 8 umfasst.

13. Verfahren zur Herstellung einer Ferninfrarot-Strahlungszusammensetzung mit hervorragenden aufladungseliminierenden Eigenschaften, wobei das Verfahren umfasst: Mischen von Aluminiumoxid und von mindestens einem ausgewählt aus Siliziumoxid und Titanoxid einzeln mit Platin oder einer Platin-Verbindung, welche(s) in einer kolloidalen Form dispergiert ist, um den jeweiligen Teilchen zu ermöglichen, dass sie Platin darauf abgelagert aufweisen; Rühren und Mischen der resultierenden Teilchen, die Platin darauf abgelagert aufweisen; und anschließend Mischen in das Pulver aus Silber oder einer Silber-Verbindung, um eine Zusammensetzung zu bilden.

## Revendications

1. Composition à rayonnement infrarouge lointain possédant des propriétés antistatiques excellentes, ladite composition comprenant (i) de l'alumine, (ii) au moins l'un choisi parmi la silice et l'oxyde de titane, (iii) au moins un élément ou un composé choisi parmi le platine, un composé du platine, le palladium, un composé du palladium, l'iridium, un composé de l'iridium, le rhodium et un composé du rhodium, et (iv) au moins l'un choisi parmi l'argent et un composé de l'argent.

2. Composition à rayonnement infrarouge lointain possédant des propriétés antistatiques excellentes selon la revendication 1, qui comprend 20 à 60 % en poids dudit composant (i), 40 à 80 % en poids dudit composant (ii), 0,0005 à 0,010 % en poids dudit composant (iii), et 0,1 à 10 % en poids dudit composant (iv).

3. Composition à rayonnement infrarouge lointain possédant des propriétés antistatiques excellentes selon la revendication 1 ou 2, dans laquelle ledit composant (ii) est l'oxyde de titane.

4. Composition à rayonnement infrarouge lointain possédant des propriétés antistatiques excellentes selon l'une quelconque des revendications 1 à 3, dans laquelle ledit composant (iii) est le platine ou un composé du platine.

5. Matériau à rayonnement infrarouge lointain possédant des propriétés antistatiques excellentes, ledit matériau comprenant la composition selon l'une quelconque des revendications 1 à 4 qui est sous la forme de poudre.

6. Matériau possédant des propriétés anti-statiques excellentes et des propriétés de rayonnement infrarouge lointain excellentes, dans lequel la composition selon l'une quelconque des revendications 1 à 5 est fixée sur une surface de celui-ci ou dans des vides internes de celui-ci.

7. Mélange à rayonnement infrarouge lointain possédant des propriétés antistatiques excellentes, ledit mélange contenant :
(A) 0,1 à 25 % en poids de la composition selon l'une quelconque des revendications 1 à 5 ; et
(B) 75 à 99,9 % en poids d'un matériau polymère synthétique.

8. Fibre synthétique comprenant le mélange selon la revendication 7.

9. Matériau de structure spongieuse élastique ou matériau de panneau comprenant le mélange selon la revendication 7.

10. Produit d'habillement comprenant la fibre synthétique selon la revendication 8.

11. Produit de literie comprenant la fibre synthétique selon la revendication 8.

12. Produit d'emballage comprenant la fibre synthétique selon la revendication 8.

13. Procédé destiné à produire une composition à rayonnement infrarouge lointain possédant des propriétés antistatiques excellentes, ledit procédé consistant : à mélanger de l'alumine et au moins l'un choisi parmi la silice et l'oxyde de titane individuellement avec du platine ou un composé du platine dispersé dans une forme colloïdale afin de permettre aux particules respectives d'avoir du platine déposé dessus ; à agiter et à mélanger les particules résultantes ayant du platine déposé dessus ; puis à mélanger dans la poudre d'argent ou d'un composé de l'argent pour former une composition.
